# EUROPEAN PATENT SPECIFICATION

(11) **EP 3 272 365 B1**
(45) Date of publication and mention of the grant of the patent: **05.08.2026**
(21) Application number: 17180548.4
(22) Date of filing: 10.07.2017
(51) Int. Cl.: A61L 2/22, A61L 9/14, B01D 46/00

(54) **A STERILIZATION SYSTEM FOR AN ASEPTIC CONTAINERS PRODUCTION MACHINE**
STERILISATIONSANLAGE ZUR HERSTELLUNG KEIMFREIER BEHÄLTER
SYSTÈME DE STÉRILISATION POUR FABRIQUER UN RÉCIPIENT SANS GERMES

(30) Priority: 11.07.2016 IT 201600072033
(43) Date of publication of application: 24.01.2018
(73) Proprietor: IPI S.r.l., 06132 Perugia (PG) (IT)
(72) Inventor: BIRELLO, Fabio, I-40135 Bologna BO (IT); CRISTOFORI, Alessandro, I-40124 Ferrara FE (IT); FINI, Fabio, I-40037 Sasso Marconi BO (IT); VITALI, Antonio, I-40062 Molinella BO (IT); NEGRINI, Stefano, I-40012 Calderara di Reno BO (IT)
(74) Representative: Buzzi, Notaro & Antonielli d'Oulx S.p.A.

(56) References cited:
- EP-A1- 3 034 151
- EP-A2- 0 815 919
- WO-A1-2004/016988
- DE-A1- 102007 017 750
- DE-A1- 102007 062 475

## Description

The present invention falls within the technical field of the machines for producing aseptic containers. In particular, the invention relates to a sterilization system for an aseptic containers production machine.

The aseptic containers (for example used for pourable food products such as fruit juices, sauces of various kinds, etc.) are obtained starting from a continuous band of packaging material formed by a plurality of layers of different materials coupled one to another (generally paper, aluminium and heat-seal plastic material, for example polyethylene), that is processed in a dedicated automatic machine.

A machine intended for making such containers comprises a feeding station, wherein a band of packaging material is fed, and in particular is unwound from a reel.

The machine comprises, downstream with respect to the feeding station, a sterilization station at which the band is contacted with a sterilization substance (generally hydrogen peroxide), for example by supplying the latter on the band.

For this purpose, the machine comprises a sterilization system comprising in turn a chamber containing a first group of filters and a second group of filters, which are disposed in cascade, at which airflow is passed. The system further comprises supplying means that supply the sterilization substance on the second group of filters. Thus, the airflow containing the sterilization substance, exiting from the second group of filters, is contacted with the packaging material band.

The packaging material band is then closed to form a continuous tube, which is filled with the product to be contained.

Finally, individual filled and sealed wrappers that will be handled in dedicated stations conferring their final shape (generally a parallelepiped shape) are obtained from the continuous tube.

However, the sterilization system described above shows some drawbacks.

In fact, the supply is made in a localized part of the second group of filters: in this way, a uniform distribution of the sterilization substance in the airflow is not obtained. This can lead to poor efficiency of the sterilization system itself. Furthermore, such a supply of the sterilization substance on the second group of filters can involve damaging the latter.

Documents WO 2004/016988, EP 3 034 151 A1, EP 0 815 919 A2 and DE 10 2007062475 A1 disclose certain prior art of possible interest as technical background. It is an object of the present invention to overcome the above mentioned drawbacks. Such an object is achieved by providing a sterilization system in accordance with the attached claims.

Advantageously, the sterilization system provided by the invention avoids damaging the filters and ensures a uniform distribution of the sterilization substance in the airflow.

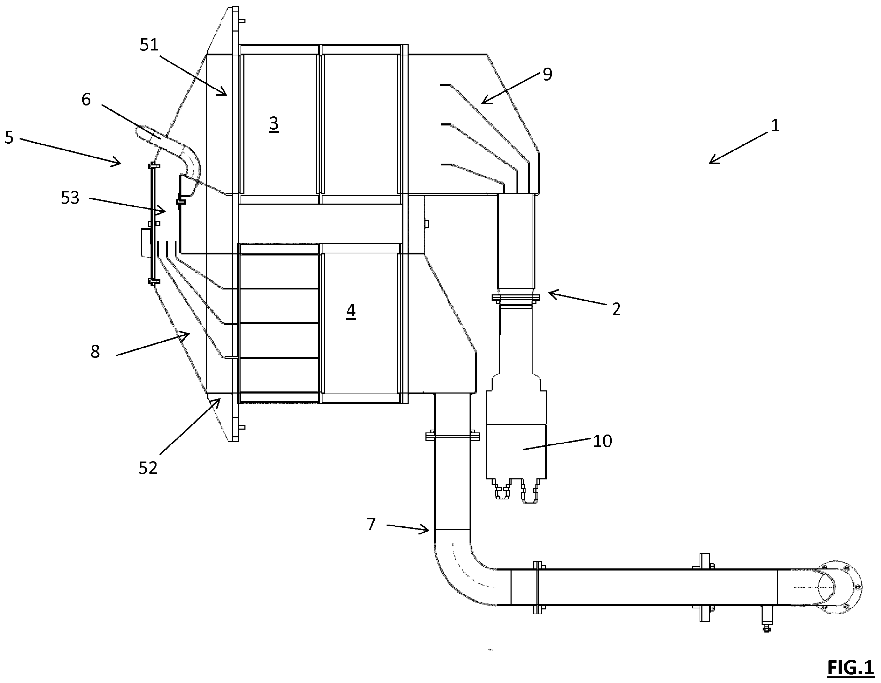

Further advantages of the invention will be made apparent in the following description, with the aid of attached figure 1, depicting in a schematic and exemplary manner a section of a sterilization system according to the invention.

With reference to attached figure 1, with the numeral reference 1 has been depicted a sterilization system for a machine for the production of aseptic containers according to the present invention.

The system 1 comprises: an airflow inlet section 2 for the entrance of an airflow; a first filtering group 3 disposed downstream with respect to the airflow inlet section 2 and communicating with the latter for receiving the airflow; and a second filtering group 4 disposed downstream with respect to the first filtering group 3 for receiving the airflow exiting from the first filtering group 3.

The system 1 further comprises: a connection zone 5 connecting the first filtering group 3 to the second filtering group 4; supplying means 6 for supplying a sterilization substance; and an airflow exiting section 7 disposed downstream with respect to the second filtering group 4 and communicating with the latter for allowing the exit of the airflow containing the sterilization substance.

In particular, the connection zone 5 defines an airflow passage conduit comprising: a first section 51 communicating with the first filtering group 3; a second section 52 communicating with the second filtering group 4; and a third section 53 interposed between the first section 51 and the second section 52; in detail, the third section 53 is smaller than the first section 51 and the second section 52. In other words, the airflow passage conduit defined by the connection zone 5 has a constriction along the relative development (indeed, at the mentioned third section 53).

Furthermore, the supplying means 6 (e.g. supplying nozzles/injectors) are designed for supplying the sterilization substance at the third section 53 of the connection zone 5 (i.e. in the part interested by the mentioned constriction), optimizing the sterilization substance distribution in the airflow.

Advantageously, the sterilization system 1 according to the invention allows a uniform distribution of the sterilization substance in the airflow. In fact, the connection zone 5 defines an airflow passage conduit along which the airflow exiting from the first air-filtering group 3 is intended to pass, i.e. it defines a path for the airflow itself.

Along such a path, and in particular at the third section 53 of the connection zone 5, constricted with respect to the first section 51 and the second section 52 among which is interposed, the sterilization substance is supplied. Consequently, advantageously, the supply of the sterilization substance is necessarily taking place in the airflow uniformly, the latter inevitably passing at the third section 53.

In other words, the shape of the airflow passage conduit formed by the connection zone 5 (i.e. the relative first section 51, second section 52 and third section 53) is fluid-dynamically designed to optimize the uniform diffusion of the sterilization substance in the airflow.

According to a shown preferred embodiment, the sterilization system 1 according to the invention further comprises a first channel group 8 at the connection zone 5, to distribute the airflow containing the sterilization substance toward the second filtering group 4.

Advantageously, the first channel group 8 optimizes the distribution of the sterilization substance, the latter being uniformly distributed in the airflow, toward the second filtering group 4. Thus, the first channel group 8 has the collector function.

The system 1 can further comprise a second channel group 9 disposed between the airflow inlet section 2 and the first filtering group 3, to distribute the airflow toward the first filtering group 3 (see figure 1 attached). Such a second channel group 9 optimizes the distribution of the airflow inside the first filtering group 3.

Preferably, the first filtering group 3 and the second filtering group 4 are mutually aligned.

For example, with reference to the figure, the first filtering group 3 and the second filtering group 4 are placed side by side one to other. Alternatively, the first filtering group 3 and the second filtering group 4 can be positioned in a different way.

In accordance with the depicted embodiment, the airflow passage conduit defined by the connection zone 5 substantially has a "C profile" development. Such a configuration ensures a particularly compact overall footprint to the whole system 1. Furthermore, the system 1 can comprise: a fan (not shown) for feeding the airflow and disposed upstream with respect to the airflow inlet section 2; and a heater 10 interposed between the fan and the airflow inlet section 2, for heating the airflow fed by the fan so that the latter reaches the conditions suitable for sterilizing the packaging material band and for ensuring its drying.

Preferably, the first filtering group 3 and/or the second filtering group 4 comprise a plurality of HEPA filters and/or ULPA filters (with high efficiency).

For example, the sterilization substance supplied by the supplying means 5 is hydrogen peroxide.

The invention further relates to a machine (not shown) for producing aseptic containers, for example for pourable food products such as fruit juices, sauces of various kinds, etc.

The machine comprises: a feeding station, which feeds a packaging material band, and a sterilization station disposed downstream with respect to the feeding station, in which the packaging material band is sterilized, the sterilization station comprising a sterilization system 1 according to any one of the embodiments described above. The machine further comprises: at least a welding station disposed downstream with respect to the sterilization station, in which the packaging material band is closed to form a continuous tube of packaging material; and a filling station, in which the tube of packaging material is filled with a product.

## Claims

1. A sterilization system (1) for an aseptic containers production machine, comprising:
an airflow inlet section (2) for the entrance of an airflow;
a first filtering group (3), disposed downstream with respect to the airflow inlet section (2) for receiving the airflow;
a second filtering group (4), disposed downstream with respect to the first filtering group (3) for receiving the airflow exiting from the first filtering group (3);
a connection zone (5), connecting the first filtering group (3) to the second filtering group (4);
supplying means (6) for supplying a sterilization substance; and
an airflow exiting section (7), disposed downstream with respect to the second filtering group (4) for allowing the exit of the airflow containing the sterilization substance;
**characterized in that**:
the connection zone (5) defines an airflow passage conduit comprising: a first section (51) communicating with the first filtering group (3); a second section (52) communicating with the second filtering group (4); and a third section (53) interposed between the first section (51) and the second section (52), having a flow section smaller compared to the first section (51) and the second section (52);
and **in that** the supplying means (6) are designed for supplying the sterilization substance at the third section (53) of the connection zone (5), optimizing the sterilization substance distribution in the airflow.

2. The sterilization system (1) according to claim 1, further comprising a first channel group (8) at the connection zone (5), to distribute the airflow containing the sterilization substance toward the second filtering group (4).

3. The sterilization system (1) according to anyone of the preceding claims, further comprising a second channel group (9) disposed between the airflow inlet section (2) and the first filtering group (3), to distribute the airflow toward the first filtering group (3).

4. The sterilization system (1) according to anyone of the preceding claims, in which the first filtering group (3) and the second filtering group (4) are mutually aligned.

5. The sterilization system (1) according to the preceding claim, in which the airflow passage conduit defined by the connection zone substantially has a "C profile" development.

6. The sterilization system (1) according to anyone of the preceding claims, further comprising: a fan, disposed upstream with respect to the airflow inlet section (2), for supplying the airflow; a heater (10) interposed between the fan and the airflow inlet section (2), for heating the airflow supplied by the fan.

7. The sterilization system (1) according to anyone of the preceding claims, in which the first filtering group (3) and/or the second filtering group (4) comprises a plurality of HEPA filters and/or ULPA filters.

8. A machine for producing aseptic containers comprising: a feeding station, which feeds a packaging material band; a sterilization station, disposed downstream with respect to the feeding station, in which the packaging material band is sterilized; said sterilization station comprising a sterilization system (1) according to anyone of the preceding claims; at least one welding station, disposed downstream with respect to the sterilization station, in which the packaging material band is closed to form a continuous tube of packaging material; and a filling station in which the tube of packaging material is filled with a product.

## Patentansprüche

1. Sterilisationssystem (1) für eine aseptische Behälterproduktionsmaschine, umfassend:
einen Luftstrom-Einlassabschnitt (2) für den Eintritt eines Luftstroms;
eine erste Filtergruppe (3), die stromabwärts in Bezug auf den Luftstrom-Einlassabschnitt (2) angeordnet ist, um den Luftstrom zu empfangen;
eine zweite Filtergruppe (4), die stromabwärts in Bezug auf die erste Filtergruppe (3) angeordnet ist, um den aus der ersten Filtergruppe (3) austretenden Luftstrom zu empfangen;
eine Verbindungszone (5), die die erste Filtergruppe (3) mit der zweiten Filtergruppe (4) verbindet;
Zuführmittel (6) zum Zuführen einer Sterilisationssubstanz; und
einen Luftstrom-Auslassabschnitt (7), der stromabwärts in Bezug auf die zweite Filtergruppe (4) angeordnet ist, um den Austritt des die Sterilisationssubstanz enthaltenden Luftstroms zu ermöglichen;
**dadurch gekennzeichnet, dass**:
die Verbindungszone (5) eine Luftstrom-Durchgangsleitung definiert, umfassend: einen ersten Abschnitt (51), der mit der ersten Filtergruppe (3) in Verbindung steht; einen zweiten Abschnitt (52), der mit der zweiten Filtergruppe (4) in Verbindung steht; und einen dritten Abschnitt (53), der zwischen dem ersten Abschnitt (51) und dem zweiten Abschnitt (52) angeordnet ist und einen im Vergleich zum ersten Abschnitt (51) und zum zweiten Abschnitt (52) kleineren Strömungsquerschnitt aufweist;
und dass die Zuführmittel (6) dazu ausgelegt sind, die Sterilisationssubstanz am dritten Abschnitt (53) der Verbindungszone (5) zuzuführen, wodurch die Verteilung der Sterilisationssubstanz im Luftstrom optimiert wird.

2. Sterilisationssystem (1) nach Anspruch 1, ferner umfassend eine erste Kanalgruppe (8) an der Verbindungszone (5), um den die Sterilisationssubstanz enthaltenden Luftstrom zur zweiten Filtergruppe (4) zu verteilen.

3. Sterilisationssystem (1) nach einem der vorhergehenden Ansprüche, ferner umfassend eine zweite Kanalgruppe (9), die zwischen dem Luftstrom-Einlassabschnitt (2) und der ersten Filtergruppe (3) angeordnet ist, um den Luftstrom zur ersten Filtergruppe (3) zu verteilen.

4. Sterilisationssystem (1) nach einem der vorhergehenden Ansprüche, bei dem die erste Filtergruppe (3) und die zweite Filtergruppe (4) zueinander ausgerichtet sind.

5. Sterilisationssystem (1) nach dem vorhergehenden Anspruch, bei dem der durch die Verbindungszone definierte Luftstrom-Durchgangskanal im Wesentlichen eine "C-Profil"-Entwicklung aufweist.

6. Sterilisationssystem (1) nach einem der vorhergehenden Ansprüche, ferner umfassend: ein Gebläse, das stromaufwärts in Bezug auf den Luftstrom-Einlassabschnitt (2) angeordnet ist, um den Luftstrom zuzuführen; eine Heizvorrichtung (10), die zwischen dem Gebläse und dem Luftstrom-Einlassabschnitt (2) angeordnet ist, um den vom Gebläse zugeführten Luftstrom zu erwärmen.

7. Sterilisationssystem (1) nach einem der vorhergehenden Ansprüche, bei dem die erste Filtergruppe (3) und/oder die zweite Filtergruppe (4) eine Vielzahl von HEPA-Filtern und/oder ULPA-Filtern umfasst.

8. Maschine zur Herstellung aseptischer Behälter, umfassend: eine Zuführstation, die ein Verpackungsmaterialband zuführt; eine Sterilisationsstation, die stromabwärts in Bezug auf die Zuführstation angeordnet ist, in der das Verpackungsmaterialband sterilisiert wird; wobei die genannte Sterilisationsstation ein Sterilisationssystem (1) nach einem der vorhergehenden Ansprüche umfasst; mindestens eine Schweißstation, die stromabwärts in Bezug auf die Sterilisationsstation angeordnet ist, in der das Verpackungsmaterialband geschlossen wird, um einen kontinuierlichen Schlauch aus Verpackungsmaterial zu bilden; und eine Füllstation, in der der Schlauch aus Verpackungsmaterial mit einem Produkt gefüllt wird.

## Revendications

1. Système de stérilisation (1) pour une machine de production de récipients aseptiques, comprenant :
une section d'entrée de flux d'air (2) pour l'entrée d'un flux d'air ;
un premier groupe de filtration (3), disposé en aval par rapport à la section d'entrée de flux d'air (2) pour recevoir le flux d'air ;
un deuxième groupe de filtration (4), disposé en aval par rapport au premier groupe de filtration (3) pour recevoir le flux d'air sortant du premier groupe de filtration (3) ;
une zone de raccordement (5), reliant le premier groupe de filtration (3) au deuxième groupe de filtration (4) ;
des moyens d'alimentation (6) pour alimenter une substance de stérilisation ; et
une section de sortie de flux d'air (7), disposée en aval par rapport au deuxième groupe de filtration (4) pour permettre la sortie du flux d'air contenant la substance de stérilisation ;
**caractérisé en ce que** :
la zone de raccordement (5) définit un conduit de passage de flux d'air comprenant : une première section (51) communiquant avec le premier groupe de filtration (3) ; une deuxième section (52) communiquant avec le deuxième groupe de filtration (4) ; et une troisième section (53) interposée entre la première section (51) et la deuxième section (52), ayant une section d'écoulement plus petite par rapport à la première section (51) et à la deuxième section (52) ;
et **en ce que** les moyens d'alimentation (6) sont conçus pour alimenter la substance de stérilisation au niveau de la troisième section (53) de la zone de raccordement (5), optimisant la distribution de la substance de stérilisation dans le flux d'air.

2. Système de stérilisation (1) selon la revendication 1, comprenant en outre un premier groupe de canaux (8) au niveau de la zone de raccordement (5), pour distribuer le flux d'air contenant la substance de stérilisation vers le deuxième groupe de filtration (4).

3. Système de stérilisation (1) selon l'une quelconque des revendications précédentes, comprenant en outre un deuxième groupe de canaux (9) disposé entre la section d'entrée de flux d'air (2) et le premier groupe de filtration (3), pour distribuer le flux d'air vers le premier groupe de filtration (3).

4. Système de stérilisation (1) selon l'une quelconque des revendications précédentes, dans lequel le premier groupe de filtration (3) et le deuxième groupe de filtration (4) sont mutuellement alignés.

5. Système de stérilisation (1) selon la revendication précédente, dans lequel le conduit de passage de flux d'air défini par la zone de raccordement présente sensiblement un développement en « profil en C ».

6. Système de stérilisation (1) selon l'une quelconque des revendications précédentes, comprenant en outre : un ventilateur, disposé en amont par rapport à la section d'entrée de flux d'air (2), pour alimenter le flux d'air ; un dispositif de chauffage (10) interposé entre le ventilateur et la section d'entrée de flux d'air (2), pour chauffer le flux d'air fourni par le ventilateur.

7. Système de stérilisation (1) selon l'une quelconque des revendications précédentes, dans lequel le premier groupe de filtration (3) et/ou le deuxième groupe de filtration (4) comprend une pluralité de filtres HEPA et/ou de filtres ULPA.

8. Machine pour produire des récipients aseptiques comprenant : un poste d'alimentation, qui alimente une bande de matériau d'emballage ; un poste de stérilisation, disposé en aval par rapport au poste d'alimentation, dans lequel la bande de matériau d'emballage est stérilisée ; ledit poste de stérilisation comprenant un système de stérilisation (1) selon l'une quelconque des revendications précédentes ; au moins un poste de soudage, disposé en aval par rapport au poste de stérilisation, dans lequel la bande de matériau d'emballage est fermée pour former un tube continu de matériau d'emballage ; et un poste de remplissage dans lequel le tube de matériau d'emballage est rempli avec un produit.
